Europäisches Patentamt

European Patent Office

Office européen des brevets

(19)

(11) Numéro de publication : **0 086 709**
**B1**

(12) **FASCICULE DE BREVET EUROPÉEN**

(45) Date de publication du fascicule du brevet :
03.12.86

(51) Int. Cl.⁴ : **G 01 T 1/29**

(21) Numéro de dépôt : 83400280.0

(22) Date de dépôt : 09.02.83

(54) **Procédé d'obtention d'images tridimensionnelles d'un objet, dispositif de mise en œuvre de ce procédé et application du procédé et du dispositif à la tomographie d'un organe.**

(30) Priorité : 17.02.82 FR 8202632

(43) Date de publication de la demande :
24.08.83 Bulletin 83/34

(45) Mention de la délivrance du brevet :
03.12.86 Bulletin 86/49

(84) Etats contractants désignés :
DE GB IT NL

(56) Documents cités :
FR-A- 2 447 558
GB-A- 2 083 970
US-A- 4 302 675
ELECTRONIQUE APPLICATIONS, no. 22, pages 71-74, février/mars 1982, Paris, FR. J. TREMOLIERES: "La tomographie d'émission cardiaque: une nouvelle approche de la dynamique cardiaque"
IEEE TRANSACTIONS ON NUCLEAR SCIENCE, vol. NS25, no. 1, février 1978, pages 196-201, IEEE, New York, USA C.B. LIM et al.: "A 3-D iterative reconstruction method for stationary planar positron cameras"

(73) Titulaire : **COMMISSARIAT A L'ENERGIE ATOMIQUE Etablissement de Caractère Scientifique Technique et Industriel**
31/33, rue de la Fédération
F-75015 Paris (FR)

(72) Inventeur : **Le Coq, Gérard**
**11 Allée Traversière**
**F-94260 Fresnes (FR)**
Inventeur : **Soussaline, Françoise**
**205 Bis Bld Raspail**
**F-75014 Paris (FR)**

(74) Mandataire : **Mongrédien, André et al**
**c/o BREVATOME 25, rue de Ponthieu**
**F-75008 Paris (FR)**

## 0 086 709

### Description

La présente invention concerne un procédé d'obtention d'images tridimensionnelles d'un objet ainsi que l'application de ce procédé à la tomographie d'un organe. Elle s'applique plus particulièrement à la reconstruction d'images tridimensionnelles d'un organe du corps humain, à partir de la détection de rayonnements gamma provenant d'un traceur injecté dans l'organe. Elle peut également s'appliquer à la reconstruction d'images tridimensionnelles à partir de la détection de rayonnements issus d'un objet ou d'un organe.

On sait reconstituer la distribution radioactive d'un objet volumique, selon une succession de plans présentant des orientations quelconques par rapport à cet objet. L'acquisition des données initiales est fondée sur la mesure, à l'aide d'une caméra à scintillation sensible aux rayonnements gamma par exemple, des photons émis par un traceur radioactif injecté dans l'organe ou l'objet que l'on souhaite examiner. Cette mesure est réalisée lors d'une rotation de 360° de la caméra, autour d'un grand axe de l'organe ou de l'objet que l'on souhaite examiner. On sait, pour chacun des angles de rotation de la caméra autour de l'objet ou de l'organe, obtenir une image dite scintigraphique de celui-ci. Généralement, la caméra utilisée est une caméra à scintillation dont le collimateur est constitué par une plaque de plomb percée d'un grand nombre de trous cylindriques ou hexagonaux, à axes parallèles, donnant accès à un détecteur de rayonnements, un ensemble de trous du collimateur se trouve en regard d'une zone élémentaire de détection fournissant un signal de détection en présence d'un rayonnement. Ce collimateur permet d'éliminer les photons qui sont émis dans des diections qui ne sont pas perpendiculaires au plan du détecteur. L'image scintigraphique obtenue résulte de la somme de photons issus de cellules élémentaires de l'organe ou de l'objet qui émettent des rayonnements dans des directions perpendiculaires au plan de la caméra et donc parallèles aux canaux du collimateur. Dans ces directions, les cellules élémentaires sont alignées. En fait, l'obtention de chaque image scintigraphique, pour chaque position de la caméra, résulte des rayonnements émis par des cellules élémentaires émettrices qui sont situées sur des droites perpendiculaires au plan de la caméra. Pour chacune des positions angulaires de la caméra autour de l'organe à examiner, les signaux électriques issus du détecteur sont proportionnels au nombre de photons reçus dans des directions perpendiculaires au plan de la caméra. Selon les différentes zones élémentaires de détection, ces signaux électriques sont numérisés et enregistrés dans une mémoire de type magnétique par exemple, pour être utilisés dans la reconstruction des images de l'organe à examiner, pour les différentes positions angulaires du plan des détecteurs par rapport à l'organe ou à l'objet à examiner.

Les signaux numérisés enregistrés en mémoire sont triés en fonction de chaque position angulaire du plan des détecteurs autour de l'organe, pour une rotation de 360° de ce plan. Les valeurs numériques sont traitées par un algorithme spécialisé, de manière à reconstituer la répartition radioactive du traceur contenu dans l'organe ou l'objet, selon une méthode dite de « reconstruction ». Pour obtenir des images tomographiques dans différents plans de coupes perpendiculaires au plan du détecteur, on essaie habituellement d'effectuer un minimum de traitements des signaux issus des détecteurs, à l'aide d'un miniordinateur, sans prendre en compte de façon précise, les limitations physiques liées au système de détection des rayonnements, c'est-à-dire les limitations liées à la caméra. On ne prend pas non plus en compte l'effet de l'autoabsorption des rayonnements à l'intérieur de l'organe ou de l'objet à examiner. Toutes ces approximations sont préjudiciables à la qualité des images obtenues. Ce phénomène d'autoabsorption est en fait, pour l'énergie des rayonnements gamma couramment utilisée en médecine nucléaire, la cause la plus importante de dégradation des données quantitatives qu'il est nécessaire de traiter pour obtenir une image correcte du corps à examiner. Un système de ce type est décrit par exemple dans le brevet GB-A-2 083 970.

L'invention a pour but de remédier à ces inconvénients et notamment de permettre l'obtention d'images tridimensionnelles d'un objet, à partir de rayonnements émis par un traceur contenu dans cet objet. En tenant compte de l'atténuation des rayonnements dans cet objet ou cet organe, ainsi que de la géométrie des détecteurs de rayonnements, on assure selon l'invention, une convergence rapide des résultats des traitements correctifs des valeurs numériques correspondant aux signaux fournis par les zones de détection du détecteur de rayonnements. On rend, de plus, possibles des études quantitatives portant par exemple sur l'absorption des rayonnements émis par les cellules élémentaires de l'objet ou de l'organe, pour différents plans de coupe, les images tridimensionnelles étant en fait des images obtenues dans différents plans de coupes perpendiculaires au plan du détecteur.

L'invention vise un procédé d'obtention d'images tridimensionnelles d'un objet à partir des rayonnements émis par un traceur contenu dans cet objet, ces rayonnements étant reçus par certaines zones de détection d'un détecteur-plan, repérées par des numéros (n) dans ce plan du détecteur, ce détecteur étant animé d'un mouvement de rotation autour de l'objet, de manière à rester constamment tangent à un cylindre fictif de révolution (C) dont l'axe correspond à un axe de l'objet, les positions angulaires du détecteur-plan étant repérées par des angles $\theta_p$ entre un plan de référence ($P_R$) passant par l'axe de l'objet et un plan passant par l'axe de l'objet et perpendiculaire au plan du détecteur, l'objet étant fictivement découpé en cellules élémentaires d'émission de rayonnements repérées par leurs coordonnées dans un repère fixe de référence (i, j, k) l'axe k de ce repère étant parallèle à l'axe de l'objet, les

2

rayonnements reçus par certaines zones de détection étant ceux qui sont émis par les cellules élémentaires de l'objet dans une direction perpendiculaire au plan du détecteur, les zones de détection fournissant, pour chaque position angulaire $\theta_p$, des signaux électriques dont les amplitudes ont des valeurs qui dépendent des intensités des rayonnements reçus, ces valeurs d'amplitudes étant mémorisées pour obtenir les images de l'objet, dans des plans de coupe, d'ordonnées k perpendiculaires à l'axe de l'objet, caractérisé en ce qu'il consiste, pour obtenir les images de l'objet dans les plans de coupe :

— à engendrer à partir des valeurs d'amplitudes mémorisées, pour chaque plan de coupe d'ordonnées k pour toutes les positions angulaires $\theta_p$, et toutes les zones de détection de numéro n qui reçoivent des rayonnements, des valeurs de correction des valeurs de ces amplitudes, ces valeurs de correction étant obtenues à l'initialisation, à partir de valeurs d'amplitudes simulées correspondant à une image simulée de l'objet dans lequel il est supposé que la distribution du traceur est uniforme, et à partir de coefficients de correction mémorisés, relatifs à l'atténuation ($\mu$) des rayonnements dans l'objet, en fonction des coordonnées (i, j) de chaque cellule élémentaire et relatifs à la géomérie de chaque détecteur,

— à déterminer et à mémoriser pour chaque plan de coupe, pour toutes les positions angulaires ($\theta p$) et pour les zones de détection de numéros n qui reçoivent des rayonnements, les valeurs des différences respectives entre les valeurs des amplitudes des signaux fournis par ces zones et les valeurs de correction, les images correspondant aux valeurs des amplitudes des signaux fournis par les zones de détection étant disponibles pour les plans de coupe pour lesquels les valeurs des différences répondent à un critère prédéterminé de convergence, puis, lorsque les valeurs des différences ne répondent pas au critère prédéterminé, le procédé consistant alors :

— à engendrer d'autres valeurs de correction des valeurs des amplitudes des signaux, pour les plans de coupe d'ordonnées k, pour toutes les positions angulaires $\theta p$ et pour toutes les zones de détection de numéros n qui reçoivent des rayonnements, ces autres valeurs de correction étant obtenues à partir des valeurs des différences mémorisées, à partir desdits coefficients de correction, et à partir de coefficients ($\lambda$) mémorisés de filtrage de ces autres valeurs de correction,

— à effectuer pour chaque plan de coupe d'ordonnées k, pour toutes les positions angulaires ($\theta p$) et pour toutes les zones de détection recevant des rayonnements, des additions entre respectivement les valeurs d'amplitude mémorisées et les autres valeurs de correction, les valeurs des résultats de ces additions étant mémorisées, pour fournir les images correspondantes de l'objet dans les plans de coupe d'ordonnées k ou pour obtenir de nouvelles valeurs de correction, à partir des résultats de ces additions, jusqu'à convergence de ces résultats.

Selon une autre caractéristique de l'invention, les valeurs de correction sont obtenues pour chaque plan de coupe d'ordonnées k, pour toutes les positions angulaires $\theta p$ et pour toutes les zones recevant des rayonnements, par multiplication des valeurs des amplitudes simulées ou résultant des additions, avec les valeurs des coefficients de correction.

Selon une autre caractéristique, les autres valeurs de correction sont obtenues, pour chaque plan de coupe d'ordonnées k, pour toutes les positions angulaires $\theta p$ et pour toutes les zones de détection recevant des rayonnements, par multiplication desdites différences avec lesdits coefficients de correction et avec lesdits coefficients de filtrage.

Selon une autre caractéristique, le procédé consiste, pour chaque plan de coupe d'ordonnée k, pour toutes les positions angulaires $\theta p$, à déterminer et à mémoriser, préalablement à l'obtention des valeurs de correction, des coordonnées (i, j) d'un masque de l'espace extérieur aux images des coupes désirées, à déterminer les correspondances entre les numéros des zones de détection n et les coordonnées (i, j) des cellules élémentaires situées dans le masque, puis à comparer les numéros des zones de détection définies par le masque avec les numéros des zones de détection recevant des rayonnements.

Selon une autre caractéristique, le procédé consiste à utiliser un dispositif microordinateur relié à au moins une mémoire et à des moyens de visualisation d'images, pour commander la rotation du détecteur, le repérage des positions angulaires $\theta p$ autour de l'objet, les repérages des zones de détection et des coordonnées i, j, k, et pour effectuer toutes les opérations de mémorisation, de correction et de visualisation des images.

L'invention a aussi pour objet un dispositif de mise en œuvre du procédé pour l'obtention d'images tridimensionnelles d'un objet à partir des rayonnements émis par un traceur contenu dans cet objet, comprenant un détecteur-plan présentant des zones de détection repérées par des numéros n et aptes à fournir des signaux dépendant respectivement des intensités des rayonnements reçus dans une direction perpendiculaire au plan (P) du détecteur, ce détecteur étant animé d'un mouvement de rotation autour de l'objet, de manière à rester constamment tangent à un cylindre fictif (C) dont l'axe correspond à un axe de l'objet, des moyens pour commander et repérer les positions angulaires ($\theta p$) du détecteur, ces positions étant repérées entre un plan de référence ($P_R$) passant par l'axe de l'objet et un plan passant par l'axe de l'objet et perpendiculaire au plan du détecteur, l'objet étant fictivement découpé en cellules élémentaires d'émission de rayonnements repérées par leurs coordonnées dans un repère fixe de référence (i, j, k), l'axe k de ce repère étant parallèle à l'axe de l'objet, les rayonnements reçus par certaines zones de détection repérées par leurs numéros n étant ceux qui sont émis par les cellules élémentaires de l'objet dans une direction perpendiculaire au plan du détecteur, les zones de détection fournissant, pour chaque position angulaire $\theta p$, des signaux électriques dont les amplitudes ont des valeurs qui dépendent des

3

intensités des rayonnements reçus, ces valeurs d'amplitudes permettant d'obtenir les images de l'objet dans des plans de coupe, d'ordonnées k, perpendiculaires à l'axe de l'objet, caractérisé en ce qu'il comprend des moyens itératifs de traitement par convergence des valeurs des amplitudes des signaux issus des zones de détection qui ont reçu des rayonnements, ces moyens de traitement étant reliés à des moyens de visualisation pour afficher les images correspondant à chaque plan de coupe d'ordonnée k après chaque itération.

Selon une autre caractéristique, les moyens de traitement comprennent :

— des moyens de mémorisation qui, pour toutes les ordonnées k des plans de coupe et toutes les positions angulaires θp du détecteur, permettent de mémoriser :

les valeurs des amplitudes des signaux fournis par les zones de détection de numéros n, qui ont reçu des rayonnements,

les coordonnées (i, j) des cellules élémentaires susceptibles d'avoir émis des rayonnements vers ces zones de détection de numéros n,

des valeurs de références d'amplitudes de signaux, ces valeurs étant simulées ou obtenues à la suite d'un précédent traitement par le dispositif, des valeurs des signaux correspondant aux zones de détection,

des coefficients de correction à appliquer aux valeurs de référence,

au moins un coefficient de filtrage de valeurs d'amplitudes,

— des premiers moyens de multiplication des valeurs d'amplitudes de référence avec les coefficients de correction, les valeurs des résultats de ces multiplications étant enregistrées par les moyens de mémorisation,

— des moyens pour comparer les valeurs des amplitudes des signaux fournis par les zones de détection avec les valeurs de référence, les valeurs des résultats de ces différences étant enregistrées dans les moyens de mémorisation,

— des moyens de test de la convergence des valeurs des différences en fonction d'un critère prédéterminé de convergence, les moyens de visualisation étant reliés aux moyens de test pour afficher les images des coupes d'ordonnées k lorsque le critère de convergence est respecté,

— des seconds moyens de multiplication des valeurs des différences avec les coefficients de correction et le coefficient de filtrage, ces moyens intervenant lorsque le critère de convergence n'est pas respecté, les valeurs des résultats de ces multiplications étant enregistrées dans les moyens de mémorisation,

— des moyens d'addition des valeurs des résultats des multiplications avec les valeurs des amplitudes de référence, les moyens de test étant reliés aux moyens de visualisation pour commander l'affichage des images des coupes d'ordonnées k à partir des résultats des additions, lorsqu'un critère de convergence des valeurs des résultats des additions est respecté, ces moyens d'addition étant reliés aux premiers moyens de multiplication et aux moyens de mémorisation pour que lorsque le critère de convergence des valeurs des résultats des additions n'est pas respecté, les valeurs de référence fournies à ces seconds moyens de multiplication soient les valeurs des résultats des additions.

Selon une autre caractéristique, le dispositif comprend en outre des moyens de commande des moyens de mémorisation pour que ceux-ci enregistrent, pour chaque ordonnée k de plan de coupe, et pour toutes les positions angulaires θp, des coordonnées (i, j) de masquage définissant un contour limite de reconstruction de l'objet, et des moyens pour comparer les coordonnées de masquage avec les coordonnées (i, j) des cellules élémentaires ayant émis des rayonnements, pour ne prendre en considération que les zones de détections dont les numéros n correspondent à des cellules élémentaires situées dans le contenu limite de masquage.

Selon une autre caractéristique, les coefficients de correction enregistrés dans les moyens de mémorisation sont relatifs à la géométrie des zones de détection, aux angles solides sous lesquels sont vues les zones de détection recevant des rayonnements, à partir des cellules élémentaires correspondantes de l'objet, et à l'atténuation des rayonnements en fonction de la distance qui sépare une cellule élémentaire du contour de masquage.

Enfin, selon une autre caractéristique, le procédé et le dispositif de l'invention s'appliquent à la reconstruction tomographique d'images tridimensionnelles d'un organe dans lequel a été injecté un traceur émettant des rayonnements gamma.

Les caractéristiques et avantages de l'invention ressortiront de la description qui va suivre, donnés en référence aux dessins annexés dans lesquels :

la figure 1 permet de mieux comprendre le procédé de l'invention et elle représente schématiquement différentes positions angulaires d'un détecteur-plan de rayonnements, par rapport à l'organe ou de l'objet à examiner,

les figures 2a et 2b représentent un organigramme qui montre les différentes opérations intervenant dans le procédé de l'invention,

la figure 3 illustre l'influence de la géométrie des zones de détection du détecteur en fonction de la position angulaire de ce détecteur par rapport à l'organe,

la figure 4 illustre l'influence de l'angle solide sous lequel une zone de détection est vue à partir d'une cellule élémentaire de l'organe,

la figure 5 est un schéma d'un ensemble de tomographie comprenant un dispositif permettant la

mise en œuvre du procédé de l'invention,

la figure 6 est un schéma qui montre de manière plus détaillée, les moyens de traitement intervenant dans le dispositif de l'invention.

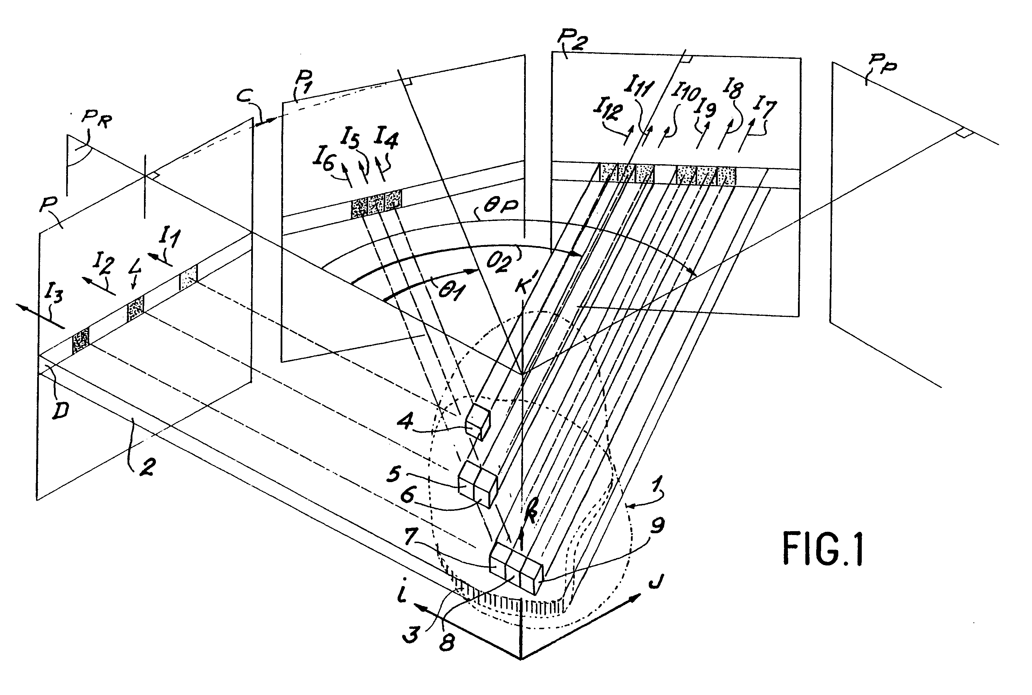

La figure 1 permet de mieux comprendre le déroulement du procédé de l'invention. On a représenté sur cette figure un objet ou un organe 1 dont on veut obtenir des images tridimensionnelles, à partir de rayonnemens émis par un traceur contenu ou injecté dans cet objet ou cet organe. On suppose que ces rayonnements peuvent être captés par des zones de détection D situées dans le plan P d'un détecteur. Chacune de ces zones de détection ne captant que les rayonnements qui sont reçus perpendiculairement au plan P grâce à un collimateur à canaux perpendiculaires à ce plan. Les zones de détection sont repérées dans ce plan par des numéros n qui peuvent être par exemple des numéros codés sous forme binaire, correspondant aux numéros des lignes et des colonnes dans lesquelles sont situées les zones de détection considérées. Selon le procédé de l'invention, le plan P lié à la caméra est animé d'un mouvement de rotation autour d'un grand axe KK' de l'objet à examiner, de manière à rester constamment tangent à un cylindre fictif de révolution C, dont l'axe correspond à celui de l'objet. Les positions angulaires successives de ce plan autour de l'objet 1 sont repérées par des angles $\theta_1$, $\theta_2$ ... $\theta_p$, mesurés entre un plan de référence $P_R$ et un plan passant par l'axe de l'objet et perpendiculaire au plan du détecteur. C'est ainsi que dans l'exemple représenté sur la figure, la position angulaire du plan P par rapport au plan de référence $P_R$ est repéré par 0, tandis que les positions successives suivantes où le plan P est représenté en $P_1$, $P_2$ ... $P_p$ sont repérées par les positions angulaires $\theta_1$, $\theta_2$ ... $\theta_p$. L'objet est supposé fictivement découpé en cellules élémentaires d'émission de rayonnements repérées par leurs coordonnées, dans un repère fixe de référence (i, j, k), dont l'axe k est parallèle au grand axe KK' de l'organe ou de l'objet. Les rayonnements reçus par le détecteur sont émis par des cellules élémentaires de l'objet dans une direction perpendiculaire au plan P du détecteur. Sur la figure, pour simplifier la représentation, on a représenté schématiquement certaines des cellules élémentaires 4, 5, 6, 7, 8, 9 contenues dans une coupe 2 de l'objet ou de l'organe ; cette coupe correspond par exemple à la portion 3 de l'objet ou de l'organe, contenue entre deux plans de coupe parallèles entre eux et perpendiculaires, d'une part, au plan P du détecteur et, d'autre part, à l'axe KK' de l'organe. Le repère i, j, k permet de repérer les ordonnées k des coupes perpendiculaires à KK'.

On a représenté schématiquement les zones D de détection situées à l'intersection de la coupe 2 avec le plan P du détecteur. On n'a représenté que quelques cellules élémentaires 4, 5, 6, 7, 8, 9, situées dans la coupe 3 et émettant des rayonnements gamma par exemple en direction de la ligne L de zone de détection D, mais il est bien évident que d'autres cellules élémentaires sont présentes. On n'a pas représenté sur cette figure, pour en faciliter la compréhension, les autres coupes de l'organe ou de l'objet 1, qui sont parallèles à la coupe 2 et qui interviennent dans le procédé ; ces coupes présentent d'autres ordonnées k. On voit sur cette figure que, dans le cas où les cellules élémentaires 4, 5 ... 9, émettrices de rayonnements sont disposées de la façon représentée et que le plan P occupe la position angulaire 0 par rapport au plan $P_R$, ce sont des zones de détection présentant des numéros n déterminés, qui fournissent des signaux $I_1$, $I_2$, $I_3$ dont les amplitudes correspondent respectivement aux contributions des intensités des rayonnements fournis par la cellule élémentaire 4, le groupe de cellules élémentaires 5, 6 et le groupe de cellules élémentaires 7 à 9. Lorsque le plan P a tourné autour de l'organe de l'objet 1, pour venir occuper la position angulaire $P_1$ repérée par $\theta_1$, ce sont d'autres zones de détection portant des numéros n déterminés et appartenant à la même ligne de zones de détection qui fournissent des signaux électriques $I_4$, $I_5$, $I_6$, dont les amplitudes comportent respectivement les contributions des intensités ou de la somme des intensités des rayonnements émis par le groupe de cellules élémentaires 4 et 9, le groupe de cellules élémentaires 6 et 8 et le groupe de cellules élémentaires 5 et 7. Il en sera de même lorsque le plan P ayant effectué une nouvelle rotation autour de l'organe 1, vient occuper la position angulaire $P_2$ repérée par $\theta_2$. Dans ce cas, ce sont des zones de détection portant également des numéros n déterminés et appartenant à la même ligne de détection que précédemment, qui fourniront des signaux $I_7$, $I_8$ ... $I_{12}$, dont les amplitudes comportent respectivement les contributions aux intensités des rayonnements émis par les cellules élémentaires 4, 5 ... 9. Le même raisonnement est applicable aux rayonnements émis par des cellules élémentaires appartenant à d'autres coupes d'ordonnées k, parallèles à la coupe 2 et non représentées sur la figure. Comme on le verra plus loin en détail, les numéros des zones de détection qui ont fourni des signaux pour chaque position angulaire $\theta_p$ du plan du détecteur autour de l'organe 1 sont enregistrés en mémoire ; ces numéros sont associés, comme on le verra plus loin, aux coordonnées i, j, k, des cellules élémentaires qui ont fourni des rayonnements en direction du détecteur. Les amplitudes des signaux fournis par certaines zones de détection sont également codées selon leurs valeurs.

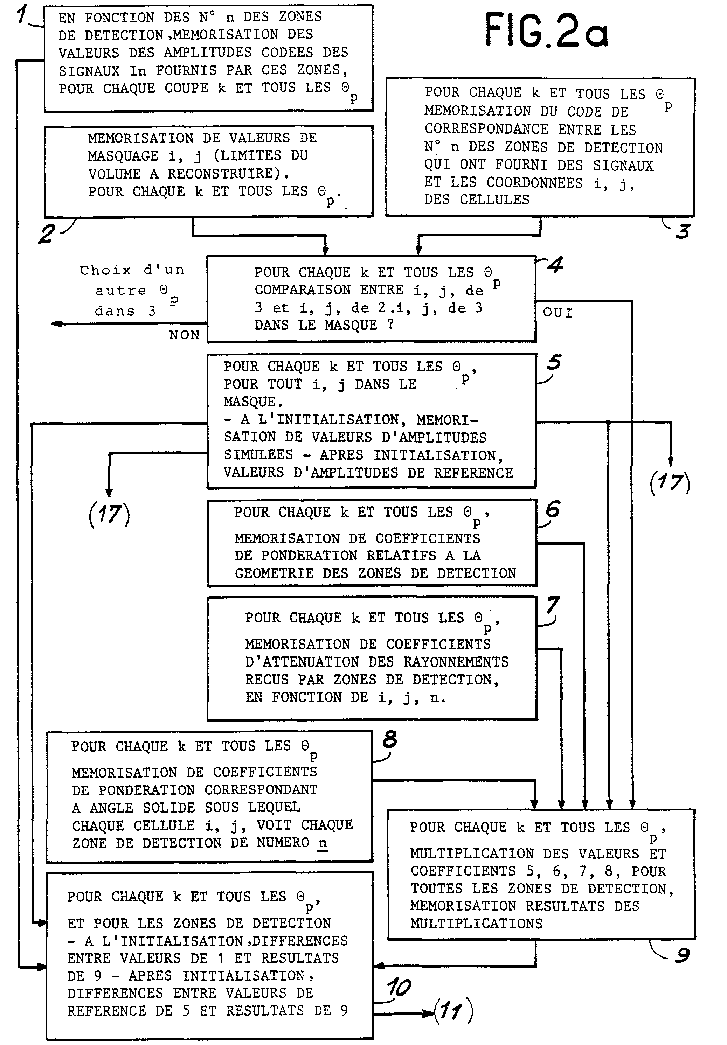

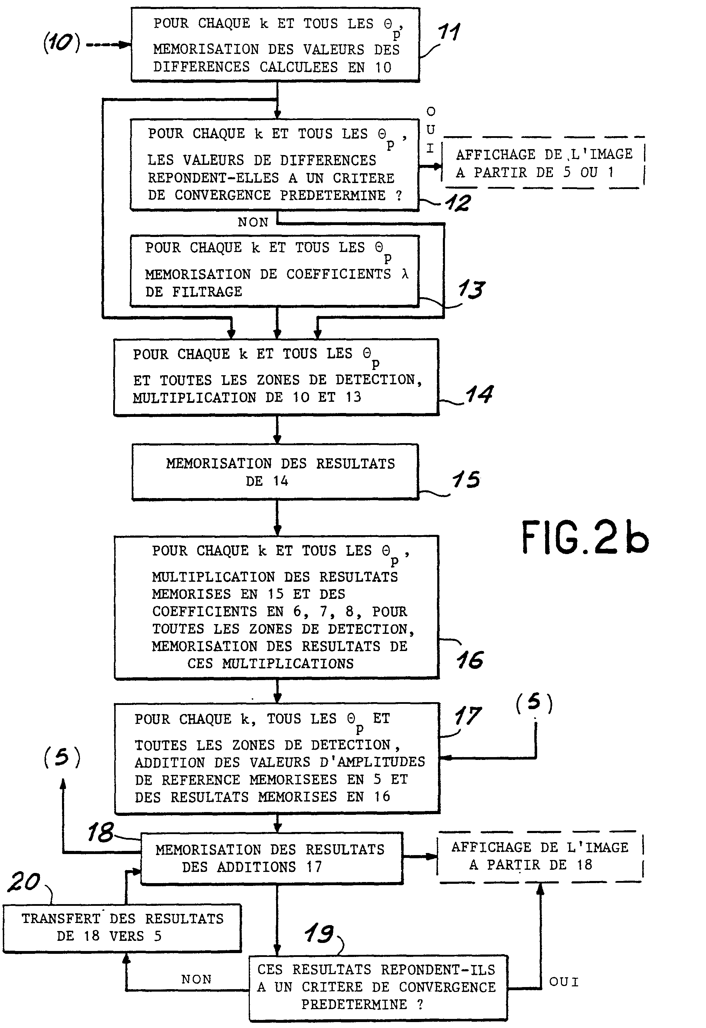

Les figures 2a et 2b représentent un organigramme qui permet de mieux comprendre le déroulement du procédé de l'invention. Cet organigramme représente les différentes étapes de ce procédé, repérées par des numéros.

Selon le procédé de l'invention et conformément à l'étape représentée en 1 sur la figure, on mémorise les valeurs codées des amplitudes des signaux $I_1$, $I_2$ ... $I_n$ fournis par les zones élémentaires de détection du détecteur qui ont reçu des rayonnements, pour chaque valeur des ordonnées k des plans de coupe et pour toutes les positions angulaires $\theta_p$ du plan du détecteur autour de l'organe 1. Ces valeurs d'amplitudes sont bien entendu associées aux numéros n des zones de détection qui ont reçu des

**0 086 709**

rayonnements, pour toutes les coupes d'ordonnées k parallèles à la coupe 2 de la figure 1. On supposera dans la suite de l'exposé que tous les traitements sont effectués pour chaque valeur de k, les différentes valeurs des positions angulaires θp étant balayées successivement pour une valeur donnée de l'ordonnée k de la coupe, avant de passer à une autre valeur de k.

Ensuite, comme indiqué en 2, on mémorise pour chaque valeur de k et toutes les positions θp, des valeurs de masquage relatives aux coordonnées limites i, j, du volume de l'organe ou de l'objet dont on veut reconstruire l'image.

L'opération suivante, référencée 3, consiste à mémoriser, pour chaque ordonnée k du plan de coupe et pour toutes les positions angulaires θp le code de correspondance entre les numéros n des zones de détection qui ont fourni des signaux $I_n$ et les coordonnées i, j, des cellules élémentaires qui sont susceptibles d'avoir émis des rayonnements vers certaines des zones de détection, dans une direction perpendiculaire à leur plan.

L'opération suivante référencée 4, consiste, pour chaque valeur de k et pour toutes les positions angulaires θp à comparer les coordonnées i, j, des cellules élémentaires qui ont émis des rayonnements et qui sont repérées à la suite de l'opération 3, avec les coordonnées i, j, de masquage définies dans l'opération 2. En fait, l'opération 4 consiste à se demander si les cellules élémentaires qui sont susceptibles d'avoir émis des rayonnements en direction de certaines zones de détection (tels que définis dans l'opération 3), sont situées à l'intérieur du volume défini par les coordonnées de masquage. Si la réponse à cette question est négative (NON), on sélectionne une autre position angulaire $\theta_p$ du plan du détecteur et on effectue la comparaison mentionnée plus haut pour d'autres valeurs des coordonnées i, j. Si au contraire, la réponse à la question posée en 4 est affirmative (OUI), on effectue l'opération 9.

Préalablement à l'opération 9, d'autres opérations de mémorisation 5, 6, 7, 8 sont à effectuer à partir des valeurs codées d'amplitudes de signaux de référence susceptibles d'être fournis par les zones de détection, pour toutes les coordonnées (i, j) des cellules élémentaires de l'objet ou de l'organe. A l'initialisation, on suppose que ces valeurs de référence sont simulées correspondant à une distribution uniforme du traceur dans l'objet. Après cette initialisation, comme on le verra plus loin en détail, ces valeurs de référence résultent d'un précédent traitement itératif de valeurs d'amplitudes.

L'opération 6 consiste à mémoriser pour toutes les zones de détection, des coefficients de pondération à appliquer aux amplitudes des signaux fournis par ces zones ; ces coefficients de pondération tiennent compte des positions relatives des zones de détection et des cellules élémentaires de l'organe ou de l'objet. En effet, comme le montre en exemple la figure 2, pour une coupe déterminée de l'organe ou de l'objet 1, la cellule élémentaire E émet des rayonnements qui sont détectés par une seule zone $Z_1$ de détection, pour une position P du plan du détecteur. Cette même cellule, pour une autre position $P_1$ du plan du détecteur, émet des rayonnements qui sont détectés, non seulement par une zone de détection $Z_3$, mais aussi par deux zones adjacentes $Z_2$ et $Z_4$. Les coefficients de pondération de l'opération 6 permettent d'apporter aux traitements des signaux issus des zones de détection, des corrections tenant compte des positions relatives des cellules et des zones de détection.

L'opération 7 consiste pour chaque ordonnée k et toutes les positions angulaires $\theta_p$ du plan de détection, à mémoriser des coefficients d'atténuation, ρ des rayonnements reçus par les zones de détection, en fonction des numéros n de ces zones et des coordonnées i, j, des cellules élémentaires susceptibles d'émettre des rayonnements en direction de ces zones de détection. En fait, ces coefficients d'atténuation sont liés à la distance qui sépare une cellule élémentaire, de la frontière de l'organe, dans une direction perpendiculaire au plan du détecteur. En effet, plus la cellule élémentaire est éloignée de la frontière de l'organe et plus l'atténuation du rayonnement qu'elle émet est importante ; ceci résulte des considérations suivantes :

Pour obtenir des données quantitatives précises en tomographie d'émission de photons gamma il est nécessaire de tenir compte de l'autoabsorption des photons dans les tissus traversés. Les données à extraire sont le volume d'un organe ou d'une cavité, la taille d'une lésion, etc...

L'autoabsorption est source d'une dégradation importante des données ; il s'agit d'un effet non linéaire pour lequel les corrections du premier ordre généralement proposées ne sont pas valables si on considère des distributions radioactives étendues et à atténuation non uniforme (cœur, foie, poumons,....).

Dans l'invention, une approche nouvelle analytique est proposée pour la reconstitution tridimensionnelle d'images. Ce problème à trois dimensions est ramené à un problème à deux dimensions : La reconstruction d'un domaine transverse à l'axe de l'objet ou de l'organe, à partir des projections, passe par la solution d'une équation intégrale qui exprime mathématiquement le problème posé ; cette intégrale est de la forme :

$$S(\vec{r}) = \iint \frac{\rho(\vec{r})}{|\vec{r} - \vec{r}'|} \left( \text{ch} \int_{|\vec{r} - \vec{r}'|} \mu \, d\rho \right) dr'$$

où $S(\vec{r})$ est une fonction déterminée à partir des projections mesurées, et $\rho(\vec{r})$ la concentration radioactive que l'on cherche à déterminer. C'est une équation intégrale de Fredholm (1$^{re}$ espèce). Cette équation peut s'écrire sous forme symbolique :

6

$$S(\vec{r}) = O_{att} \cdot \rho(\vec{r})$$

$O_{att}$ est un « opérateur tomographique » prenant en compte l'effet d'atténuation des photons dans les tissus. Une telle formulation permet d'introduire, en fait, un ensemble d'opérateurs tomographiques tenant compte de différents effets liés à la réponse physique du système de détection. Ces opérateurs peuvent être exprimés sous la forme d'opérateurs linéaires symétriques, pour lesquels une solution itérative régularisante peut être proposée. On démontre qu'une solution itérative :

$$\rho_n \cdot \vec{r} = \rho_{n-1} \cdot \vec{r} + \lambda(S(\vec{r}) - O_{att} \cdot \rho_{n-1}(\vec{r}))$$

est une solution analytique du problème posé, où $\lambda$ est un facteur de relaxation qui correspond à une norme minimale. Ce facteur est calculé de façon à gérer la convergence et l'effet régularisant sur la solution finale.

La méthode peut être qualifiée de Méthode Itérative Régularisante (MIR). La valeur de $\lambda$ peut être, dans une première étape, prise égale à une constante, mais pourrait, dans une deuxième étape, être une matrice. Cette valeur convenablement choisie permet une convergence rapide (3 à 5 itérations) et, pour ce petit nombre d'itérations, est associée à un filtrage du bruit dans la solution finale (ce filtrage sera décrit plus loin).

Cette formulation très générale du problème qui permet de trouver une solution exacte et filtrée en un petit nombre d'itérations, et qui prend en compte une atténuation des photons éventuellement distribuée dans le domaine considéré est nouvelle par rapport aux autres méthodes de reconstruction actuellement proposées qui sont soit empiriques, soit à hypothèses restrictives (domaine convexe et atténuation constante).

Une telle méthode est utilisée dans l'invention.

L'opération 8 consiste, pour chaque valeur de k et toutes les positions angulaires θp, à mémoriser des coefficients de pondération relatifs aux angles solides sous lesquels chaque cellule élémentaire du volume à reconstruire voit chaque zone de détection de rang n, en fonction des coordonnées i, j, de chacune de ces cellules élémentaires.

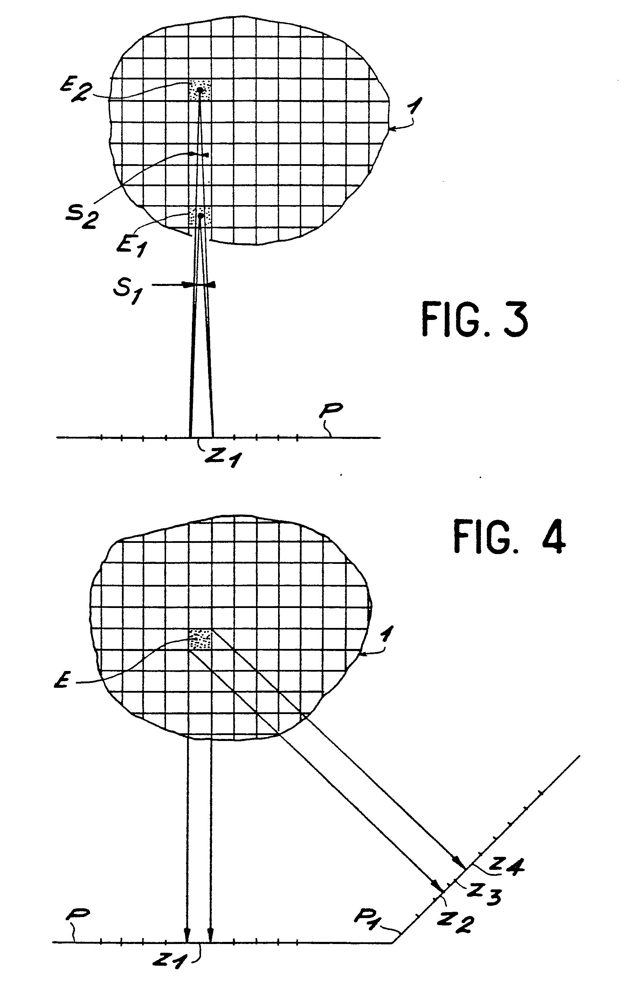

Comme le montre la figure 3, l'angle solide $(S_1)$ sous lequel la zone de détection $Z_1$ est vue à partir de la cellule élémentaire $E_1$ de l'organe 1, n'est pas le même que l'angle $S_2$, sous lequel la zone de détection $Z_1$ est vue à partir de la cellule $E_2$. Il est donc nécessaire de tenir compte de cette correction lors du traitement des signaux issus des détecteurs.

L'opération 9, de multiplication évoquée plus haut, consiste, pour chaque valeur k et pour toutes les positions angulaires $\theta_p$, à multiplier les valeurs des coefficients mémorisés en 6, 7, 8, qui peuvent être qualifiés de coefficients de correction, avec les valeurs de références définies en 5. Les résultats des multiplications sont mémorisés et constituent comme on le verra plus loin, des valeurs de correction des amplitudes des signaux $I_n$ provenant des zones de détection, et mémorisées en 1.

L'opération 10 consiste à calculer, pour chaque valeur de k, pour toutes les positions angulaires $\theta_p$, et pour toutes les zones de détection de rang n qui ont fourni des signaux, les différences entre les valeurs des amplitudes mémorisées en 1 et les valeurs des résultats des multiplications mémorisées en 9.

Les résultats des différences calculés à la suite de l'opération 10 sont mémorisés grâce à l'opération 11, pour chaque valeur de k et pour toutes les positions angulaires $\theta_p$.

L'opération 12 consiste à rechercher, pour chaque valeur de k et toutes les positions angulaires $\theta_p$, si les valeurs des différences mémorisées en 11 répondent à un critère d'arrêt ou de convergence du procédé qui peut être par exemple le fait que ces valeurs de différences sont inférieures ou supérieures à des valeurs prédéterminées. Si ces différences sont inférieures à ces valeurs prédéterminées ou si elles répondent à un autre critère d'arrêt (réponse OUI à la question 12), il est possible de procéder à l'affichage de l'image de l'objet ou de l'organe, pour un ou plusieurs plans de coupe, d'ordonnées k, à partir des valeurs des amplitudes mémorisées en 1 à l'initialisation ou en 5, après initialisation.

Si au contraire, la réponse à la question posée en 12 est NON, c'est que les valeurs des différences ne répondent pas au critère d'arrêt et sont par exemple supérieures à des valeurs prédéterminées ; il est impossible d'afficher directement l'image à partir des valeurs des amplitudes mémorisées en 1, ou des amplitudes de référence mémorisées en 5 à la suite d'un traitement, sans appliquer à ces amplitudes, d'autres valeurs de correction.

L'opération 13 est une opération dans laquelle, pour chaque valeur de k et pour toutes les positions angulaires $\theta_p$, on a mémorisé des coefficients $\lambda$ de filtrage à appliquer aux valeurs des différences mémorisées en 11, pour assurer une convergence rapide des traitements des signaux et pour filtrer les bruits à la suite de ces traitements. Ces coefficients de filtrage sont obtenus à partir de fantômes mathématiques et sont décrits plus haut.

L'opération 14 consiste à multiplier, pour chaque valeur de k et pour toutes les positions angulaires $\theta_p$, pour toutes les zones de détection de rang n, le résultat de chaque différence mémorisée en 11 et de chaque coefficient de filtrage mémorisé en 13.

L'opération 15 consiste à mémoriser les résultats des multiplications résultant de l'opération 14.

L'opération 16 consiste, pour chaque valeur de k et toutes les positions angulaires $\theta_p$, à multiplier les résultats des multiplications mémorisés en 15, avec les coefficients de pondération et d'atténuation mémorisés en 6, 7, 8, pour chaque zone de détection de rang n. Cette opération consiste aussi à mémoriser les résultats de ces dernières multiplications effectuées.

L'opération 17 consiste, pour chaque valeur de k, pour toutes les positions angulaires $\theta_p$ et pour toutes les zones de détection, à additionner les valeurs de référence mémorisées en 5, et les résultats des multiplications mémorisés en 16.

Enfin, l'opération 18 consiste à mémoriser les résultats des additions effectuées en 17. A la suite de cette mémorisation, il est possible d'afficher l'image de l'organe ou de l'objet pour un ou plusieurs plans de coupe, d'ordonnées k, si les valeurs mémorisées en 18 répondent à un critère prédéterminé de convergence (réponse OUI à la question 19). Si au contraire, cette convergence n'est pas obtenue (réponse NON à la question 19), les résultats mémorisés en 18 sont soumis aux multiplications 9, cela revient à transférer les résultats de 18 vers 5, selon l'opération référencée 20 sur la figure. Les mêmes opérations que celles qui viennent d'être décrites sont alors effectuées, à partir de l'opération 9, jusqu'à l'obtention de la convergence des résultats en 18. Cela revient donc à un traitement itératif dans lequel les valeurs en cours de traitement sont celles qui auraient permis l'affichage d'une image précédente imparfaite, pour chaque plan de coupe.

Il est bien évident que les affichages des images correspondant aux différents plans de coupe d'ordonnées k peuvent être effectués lorsque tous les traitements des signaux ont été effectués pour tous ces plans de coupe. Il est bien évident aussi que les mémorisations effectuées en 6, 7, 8, 13 sont réalisées à l'initialisation du procédé.

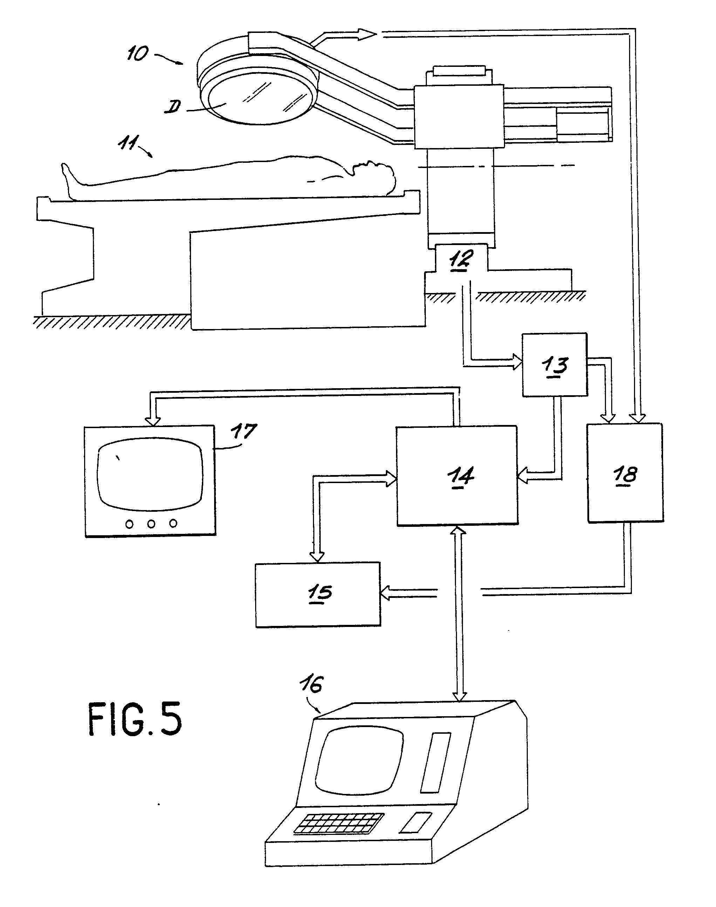

La figure 5 représente schématiquement un dispositif permettant la mise en œuvre du procédé de l'invention. Ce dispositif comprend un détecteur-plan D qui peut être par exemple un détecteur de rayonnements gamma, dans une gammacaméra 10. Les rayonnements gamma détectés, dans l'exemple considéré, sont ceux qui sont émis par des cellules élémentaires d'un organe d'un patient 11 à examiner. Le patient est disposé sur une table dont la position est réglable en hauteur et dans deux directions horizontales, tandis que le détecteur peut être déplacé en rotation autour du patient grâce à des moyens 12 d'entraînement en rotation, qui ne sont pas ici représentés en détail. Des moyens de commande 13 permettent d'une part, de commander cette rotation, et, d'autre part, de repérer les angles de rotation du détecteur-plan D, par rapport à un plan de référence qui n'est pas représenté ici. Les positions angulaires du détecteur ainsi que les valeurs d'amplitudes des signaux, repérées en fonction des numéros n des zones de détection sont fournies à un dispositif de codage 18, pour que ces valeurs d'amplitudes soient enregistrées en correspondance avec chaque position angulaire et chaque numéro de zone de détection. Les différentes mémorisations qui apparaissent dans le procédé de l'invention sont effectuées dans la mémoire 15 associée à un microcalculateur 14. Ce microcalculateur dans le dispositif de l'invention, permet d'effectuer différentes opérations de calculs de différences, d'additions ou de multiplications, mentionnés plus haut. Les différentes instructions nécessaires au fonctionnement du microcalculateur 14 peuvent être introduites dans la mémoire 15, par l'intermédiaire du microcalculateur 14, grâce à un clavier de commande 16. Il est bien évident que la mémoire 15 peut être partiellement une mémoire morte contenant des microinstructions ainsi que les valeurs des différents coefficients mentionnés plus haut. Enfin, l'affichage des images correspondant aux différents plans de coupe d'ordonnées k, est effectué sur un appareil de visualisation polychrome 17.

Il est bien évident que cet affichage n'est réalisé que lorsque les traitements de signaux ont été effectués après une rotation de 360° du plan du détecteur autour du patient.

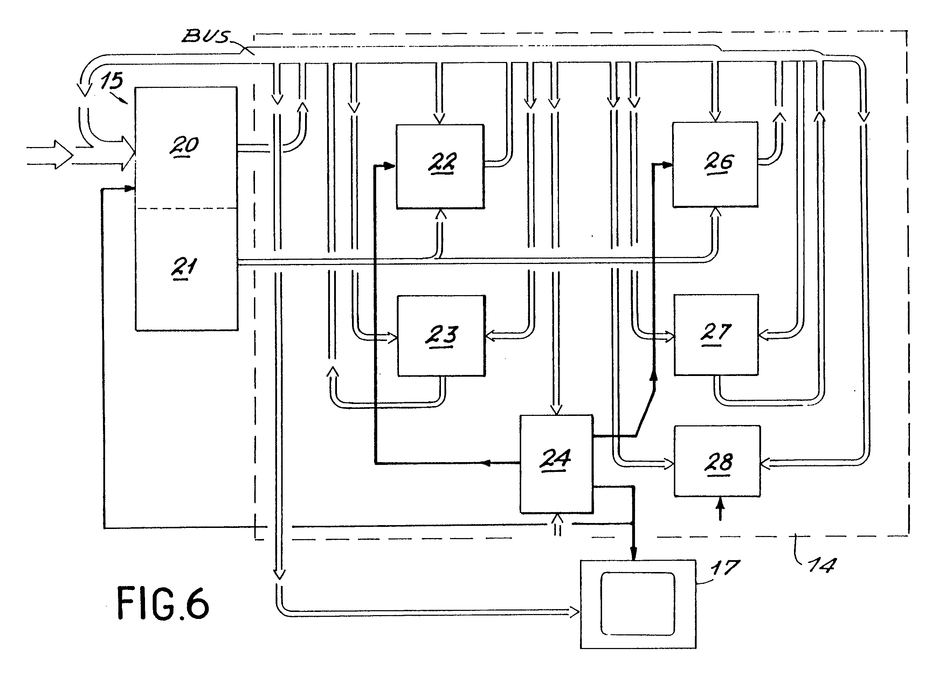

Les moyens de mémorisation constitués par la mémoire 15 associés au microcalculateur 14, constituent les moyens itératifs de traitement qui interviennent dans le dispositif de l'invention. Ces moyens vont maintenant être décrits en détail en se référant à la figure 6.

Les moyens de mémorisation 15 sont constitués par une mémoire qui peut comporter une partie vive 20 permettant d'enregistrer des données évolutives et une partie morte 21, permettant d'enregistrer des données non évolutives telles que les coefficients de correction et de filtrage qui ont été décrits plus haut : ils sont relatifs à la géométrie des zones de détection, aux angles solides sous lesquels sont vues les zones de détection recevant les rayonnements à partir des cellules élémentaires correspondantes d'objets ainsi qu'à l'atténuation des rayonnements en fonction de la distance qui sépare une cellule élémentaire du contour de masquage. Enfin, cette partie morte de la mémoire 15 permet aussi d'enregistrer le coefficient de filtrage $\lambda$ des valeurs d'amplitudes. La partie vive 20 de la mémoire 15 est mise en communication avec les différents composants du microordinateur 14 par l'intermédiaire d'un BUS. Cette partie vive permet d'enregistrer, pour toutes les ordonnées k des plans de coupe et toutes les positions angulaires $\theta p$ du détecteur, les informations suivantes :

— les valeurs des amplitudes des signaux fournis par les zones de détection de numéro n qui ont reçu des rayonnements ;

— les coordonnées i, j, des cellules élémentaires de l'objet ou de l'organe qui sont susceptibles d'avoir émis des rayonnements vers ces zones de détection de numéro n. En effet, par une étude initiale, il est possible d'établir une correspondance, pour chaque position angulaire $\theta p$ du détecteur, entre le numéro n des zones de détection, et les coordonnées i, j, k, des cellules élémentaires susceptibles d'émettre des rayonnements vers ces zones de détection, dans une direction perpendiculaire au plan du détecteur ;

— des valeurs de référence d'amplitudes de signaux ; ces valeurs comme indiqué plus haut sont simulées à l'initialisation du dispositif, ou obtenues à la suite d'un précédent traitement des valeurs des amplitudes des signaux fournis par les zones de détection,

— pour chaque plan de coupe d'ordonnée k, les coordonnées i, j, du contour de masquage des zones extérieures à l'image à obtenir.

Le microcalculateur 14 qui, avec la mémoire 15, constituent les moyens de traitement du dispositif de l'invention comprend des premiers moyens de multiplication 22 qui permettent, selon le procédé de l'invention, de multiplier les valeurs d'amplitudes de référence enregistrées dans la partie 20 de la mémoire 15, avec les coefficients de correction enregistrés dans la partie morte 21 de cette mémoire. Les valeurs des résultats de ces multiplications sont alors enregistrées dans la partie vive 20 de la mémoire 15. Ce microordinateur comprend aussi des moyens 23 qui permettent à l'initialisation, de comparer les valeurs des amplitudes des signaux fournis par les zones de détection et qui ont été enregistrés à la suite d'une rotation complète du détecteur autour de l'objet, avec les valeurs d'amplitudes simulées enregistrées dans cette mémoire. Après initialisation, la comparaison s'effectue comme on le verra plus loin, entre des valeurs d'amplitude de référence résultant d'un précédent traitement, et des valeurs d'amplitudes résultant d'un traitement en cours (valeurs des résultats en sortie des moyens de multiplication 22). Les résultats des multiplications sont enregistrés dans la mémoire 15. Des moyens de test 24 permettent, selon le procédé de l'invention, de tester la convergence des valeurs des différences fournies par les moyens de comparaison 23 et enregistrées dans la mémoire, en fonction d'un critère prédéterminé de convergence qui est traduit par des signaux de commande appliqués sur une entrée 25 des moyens de test. Les moyens de visualisation 17 sont commandés par ces moyens de test pour que, lorsque le critère de convergence des valeurs des différences est respecté, il soit possible d'afficher les images des coupes d'ordonnées k, à partir des valeurs d'amplitudes de référence qui ont été enregistrées dans la mémoire 15 et qui ont servi au calcul de ces différences. Les moyens de traitement comprennent aussi des seconds moyens de multiplication 26 qui permettent, lorsque le critère de convergence n'est pas respecté, de multiplier les valeurs des différences qui viennent d'être enregistrées dans la mémoire 15, avec les coefficients de correction mentionnés plus haut, ainsi qu'avec le coefficient λ de filtrage. Ces seconds moyens de multiplication sont commandés par les moyens de tests 24 et ils interviennent lorsque le critère de convergence n'est pas respecté. Les valeurs des résultats des multiplications sont enregistrées dans la partie vive 20 de la mémoire 15. Des moyens d'addition 27 permettent alors d'additionner les valeurs des résultats des multiplications qui viennent d'être enregistrées en mémoire avec les valeurs des amplitudes de référence enregistrées en mémoire et résultant d'un précédent traitement. Les moyens de test 24 permettent de commander l'affichage des images des coupes d'ordonnées k par les moyens de visualisation 17, lorsqu'un critère de convergence des valeurs des résultats des additions est respecté. Cet affichage est réalisé à partir des résultats de ces additions. Si au contraire, le critère de convergence n'est pas respecté, les moyens de test 24 commandent les premiers moyens de multiplication 22 pour que tous les traitements effectués par les moyens 22, 23, 24, 26, 27, 28 se reproduisent de manière identique ; dans ce cas, les moyens de multiplication 22 ne traitent pas des valeurs simulées d'amplitudes, comme à l'initialisation, mais ils multiplient les résultats des additions qui viennent d'être enregistrés, par les coefficients de correction mentionnés plus haut. Le processus de traitement se répète de manière identique jusqu'à obtenir une convergence des résultats, soit après les différences calculées par les moyens de comparaison 23, soit après les additions calculées par les moyens d'addition 27. Cette convergence plus ou moins rapide dépend en fait du coefficient de filtrage λ.

Enfin, comme indiqué plus haut, il est possible d.enregistrer dans la partie vive 20 de la mémoire 15, pour chaque ordonnée k de plan de coupe et pour toutes les positions angulaires θp, les coordonnées i, j, de masquage qui définissent dans chaque plan de coupe, le contour limite de visualisation de l'objet dans ce plan de coupe. Les moyens de traitement comprennent alors des moyens supplémentaires 28 de comparaison, qui permettent de comparer les coordonnées de masquage enregistrées dans la mémoire 15 avec les coordonnées i, j, des cellules élémentaires qui ont émis des rayonnements, ces coordonnées étant enregistrées en mémoire. Ce comparateur permet en fait d'incrémenter l'adressage de la partie vive de la mémoire 15 pour ne prendre en considération que les zones de détection dont les numéros n correspondent à des cellules élémentaires situées dans le contour limite de masquage. Lorsque après comparaison, les coordonnées i, j des cellules élémentaires qui ont reçu des rayonnements ne sont pas situées à l'intérieur du contour limite de masquage, le comparateur 28 permet d'accéder aux informations utiles au traitement, à une adresse de la mémoire qui correspond à la position angulaire θp suivante.

## Revendications

1. Procédé d'obtention d'images tridimensionnelles d'un objet à partir des rayonnements émis par un traceur contenu dans cet objet, ces rayonnements étant reçus par certaines zones de détection d'un détecteur-plan, repérées par des numéros (n) dans ce plan du détecteur, ce détecteur étant animé d'un mouvement de rotation autour de l'objet, de manière à rester constamment tangent à un cylindre fictif de révolution (C) dont l'axe correspond à un axe de l'objet, les positions angulaires du détecteur-plan étant repérées par des angles $\theta_p$ entre un plan de référence $(P_R)$ passant par l'axe de l'objet et un plan passant par l'axe de l'objet det perpendiculaire au plan du détecteur, l'objet étant fictivement découpé en cellules élémentaires d'émission de rayonnements repérées par leurs coordonnées dans un repère fixe de référence (i, j, k) l'axe k de ce repère étant parallèle à l'axe de l'objet, les rayonnements reçus par certaines zones de détection étant ceux qui sont émis par les cellules élémentaires de l'objet dans une direction perpendiculaire au plan du détecteur, les zones de détection fournissant, pour chaque position angulaire $\theta_p$, des signaux électriques dont les amplitudes ont des valeurs qui dépendent des intensités des rayonnements reçus, ces valeurs d'amplitudes étant mémorisées pour obtenir les images de l'objet, dans des plans de coupe, d'ordonnées k perpendiculaires à l'axe de l'objet, caractérisé en ce qu'il consiste, pour obtenir les images de l'objet dans les plans de coupe :

— à engendrer à partir des valeurs d'amplitudes mémorisées, pour chaque plan de coupe d'ordonnées k pour toutes les positions angulaires $\theta p$, et toutes les zones de détection de numéro n qui reçoivent des rayonnements, des valeurs de correction des valeurs de ces amplitudes, ces valeurs de correction étant obtenues à l'initialisation, à partir de valeurs d'amplitudes simulées correspondant à l'initialisation, une image simulée de l'objet dans lequel il est supposé que la distribution du traceur est uniforme, et à partir de coefficients de correction mémorisés, relatifs à l'atténuation ($\mu$) des rayonnements dans l'objet, en fonction des coordonnées (i, j) de chaque cellule élémentaire, et relatifs à la géométrie de chaque détecteur,

— à déterminer et à mémoriser pour chaque plan de coupe, pour toutes les positions angulaires ($\theta p$) et pour les zones de détection de numéros n qui reçoivent des rayonnements, les valeurs des différences respectives entre les valeurs des amplitudes des signaux fournis par ces zones et les valeurs de correction, les images correspondant aux valeurs des amplitudes des signaux fournis par les zones de détection étant disponibles pour les plans de coupe pour lesquels les valeurs des différences répondent à un critère prédéterminé de convergence, puis, lorsque les valeurs des différences ne répondent pas au critère prédéterminé, le procédé consistant alors :

— à engendrer d'autres valeurs de correction des valeurs des amplitudes des signaux, pour les plans de coupe d'ordonnées k, pour toutes les positions angulaires $\theta p$ et pour toutes les zones de détection de numéros n qui reçoivent des rayonnements, ces autres valeurs de correction étant obtenues à partir des valeurs des différences mémorisées, à partir desdits coefficients de correction, et à partir de coefficients ($\lambda$) mémorisés de filtrage de ces autres valeurs de correction,

— à effectuer pour chaque plan de coupe d'ordonnées k, pour toutes les positions angulaires ($\theta p$) et pour toutes les zones de détection recevant des rayonnements, des additions entre respectivement les valeurs d'amplitude mémorisées et les autres valeurs de correction, les valeurs des résultats de ces additions étant mémorisées, pour fournir les images correspondantes de l'objet dans les plans de coupe d'ordonnées k ou pour obtenir de nouvelles valeurs de correction, à partir des résultats de ces additions, jusqu'à convergence de ces résultats.

2. Procédé selon la revendication 1, caractérisé en ce que les valeurs de correction sont obtenues pour chaque plan de coupe d'ordonnées k, pour toutes les positions angulaires $\theta p$ et pour toutes les zones recevant des rayonnements, par multiplication des valeurs des amplitudes simulées ou résultant des additions, avec les valeurs des coefficients de correction.

3. Procédé selon la revendication 2, caractérisé en ce que les autres valeurs de correction sont obtenues, pour chaque plan de coupe d'ordonnées k, pour toutes les positions angulaires $\theta p$ et pour toutes les zones de détection recevant des rayonnements, par multiplication desdites différences avec lesdits coefficients de correction et avec lesdits coefficients de filtrage.

4. Procédé selon la revendication 3, caractérisé en ce qu'il consiste, pour chaque plan de coupe d'ordonnée k, pour toutes les positions angulaires $\theta p$, à déterminer et à mémoriser, préalablement à l'obtention des valeurs de correction, des coordonnées (i, j) d'un masque de l'espace extérieur aux images des coupes désirées, à déterminer les correspondances entre les numéros des zones de détection n et les coordonnées (i, j) des cellules élémentaires situées dans le masque, puis à comparer les numéros des zones de détection définies par le masque avec les numéros des zones de détection recevant des rayonnements.

5. Procédé selon la revendication 4, caractérisé en ce qu'il consiste à utiliser un dispositif microordinateur relié à au moins une mémoire et à des moyens de visualisation d'images, ce dispositif permettant en outre de commander la rotation du détecteur, le repérage des positions angulaires $\theta p$ autour de l'objet, les repérages des zones de détection et des coordonnées i, j, k, et pour effectuer toutes les opérations de mémorisation, de correction et de visualisation des images.

6. Dispositif d'obtention d'images tridimensionnelles d'un objet à partir des rayonnements émis par un traceur contenu dans cet objet, comprenant un détecteur-plan (D) présentant des zones de détection repérées par des numéros n et aptes à fournir des signaux dépendant respectivement des intensités des rayonnements reçus dans une direction perpendiculaire au plan (P) du détecteur, ce détecteur étant animé d'un mouvement de rotation autour de l'objet, de manière à rester constamment tangent à un cylindre fictif (C) dont l'axe correspond à un axe de l'objet, des moyens (13) pour commander et repérer les positions angulaires ($\theta p$) du détecteur, ces positions étant repérées entre un plan de référence ($P_R$) passant par l'axe de l'objet et un plan passant par l'axe de l'objet et perpendiculaire au plan du détecteur, l'objet étant fictivement découpé en cellules élémentaires d'émission de rayonnements repérées par leurs coordonnées dans un repère fixe de référence (i, j, k), l'axe k de ce repère étant parallèle à l'axe de l'objet, les rayonnements reçus par certaines zones de détection repérées par leurs numéros n étant ceux qui sont émis par les cellules élémentaires de l'objet dans une direction perpendiculaire au plan du détecteur, les zones de détection fournissant, pour chaque position angulaire θp, des signaux électriques dont les amplitudes ont des valeurs qui dépendent des intensités des rayonnements reçus, ces valeurs d'amplitudes permettant d'obtenir les images de l'objet dans des plans de coupe, d'ordonnées k, perpendiculaires à l'axe de l'objet, le dispositif comprenant aussi des moyens itératifs (14, 15) de traitement par convergence des valeurs des amplitudes des signaux issus des zones de détection qui ont reçu des rayonnements, ces moyens de traitement étant reliés à des moyens de visualisation (17) pour afficher les images de l'objet correspondant à chaque plan de coupe d'ordonnée k, après chaque itération, caractérisé en ce que les moyens de traitement comprennent :

— des moyens de mémorisation (15) qui, pour toutes les ordonnées k des plans de coupe et toutes les positions angulaires θp du détecteur, permettent de mémoriser :

les valeurs des amplitudes des signaux fournis par les zones de détection de numéros n, qui ont reçu des rayonnements,

les coordonnées (i, j) des cellules élémentaires susceptibles d'avoir émis des rayonnements vers ces zones de détection de numéros n,

des valeurs de références d'amplitudes de signaux, ces valeurs étant simulées ou obtenues à la suite d'un précédent traitement par le dispositif, des valeurs des signaux correspondant aux zones de détection,

des coefficients de correction à appliquer aux valeurs de référence,

au moins un coefficient de filtrage de valeurs d'amplitudes,

— des premiers moyens de multiplication (22) des valeurs d'amplitudes de référence avec les coefficients de correction, les valeurs des résultats de ces multiplications étant enregistrées par les moyens de mémorisation (15),

— des moyens (23) pour comparer les valeurs des amplitudes des signaux fournis par les zones de détection avec les valeurs de référence, les valeurs des résultats de ces différences étant enregistrées dans les moyens de mémorisation (15),

— des moyens de test (24) de la convergence des valeurs des différences en fonction d'un critère prédéterminé de convergence, les moyens de visualisation (17) étant reliés aux moyens de test (24) pour afficher les images des coupes d'ordonnées k lorsque le critère de convergence est respecté,

— des seconds moyens de multiplication (26) des valeurs des différences avec les coefficients de correction et le coefficient de filtrage, ces moyens intervenant lorsque le critère de convergence n'est pas respecté, les valeurs des résultats de ces multiplications étant enregistrées dans les moyens de mémorisation (15),

— des moyens d'addition (27) des valeurs des résultats des multiplications avec les valeurs des amplitudes de référence, les moyens de test (24) étant reliés aux moyens de visualisation (17) pour commander l'affichage des images des coupes d'ordonnées k, à partir des résultats des additions, lorsqu'un critère de convergence des valeurs des résultats des additions est respecté, ces moyens d'addition étant reliés aux premiers moyens de multiplication (22) et aux moyens de mémorisation (15) pour que lorsque le critère de convergence des valeurs des résultats des additions n'est pas respecté, les valeurs de référence fournies à ces premiers moyens de multiplication (22) soient les valeurs des résultats des additions.

7. Dispositif selon la revendication 6, caractérisé en ce qu'il comprend en outre des moyens de commande (16) des moyens de mémorisation (15) pour que ceux-ci enregistrent, pour chaque ordonnée k de plan de coupe, et pour toutes les positions angulaires θp, des coordonnées (i, j) de masquage définissant un contour limite de reconstruction de l'image de l'objet, et des moyens (28) pour comparer les coordonnées de masquage avec les coordonnées (i, j) des cellules élémentaires ayant émis des rayonnements, pour ne prendre en considération que les zones de détections dont les numéros n correspondent à des cellules élémentaires situées dans le contour limite de masquage.

8. Dispositif selon la revendication 7, caractérisé en ce que les coefficients de correction enregistrés dans les moyens de mémorisation (15) sont relatifs à la géométrie des zones de détection, aux angles solides sous lesquels sont vues les zones de détection recevant des rayonnements à partir des cellules élémentaires correspondantes de l'objet, et à l'atténuation des rayonnements en fonction de la distance qui sépare une cellule élémentaire du contour de masquage.

9. Application du procédé et du dispositif conformes à l'une quelconque des revendications 1 à 8 à la reconstruction tomographique d'images tridimensionnelles d'un organe dans lequel a été injecté un traceur émettant des rayonnements gamma.

## Claims

1. Process for obtaining three-dimensional images of an object from radiation emitted by a tracer contained within that object, this radiation being received by certain detection zones of a detector-plane, referenced by numbers (n) in this plane of the detector, this detector being rotated around the object in such a way that it remains constantly tangential to an imaginary cylinder of revolution (C) whose axis corresponds to an axis of the object, the angular positions of the detector-plane being referenced by

angles $\theta_p$ between a reference plane ($P_R$) passing through the axis of the object and a plane passing through the axis of the object and perpendicular to the plane of the detector, the object being divided up into imaginary elementary radiation-emitting cells referenced by their coordinates in a fixed frame of reference (i, j, k), the k axis of this frame of reference being parallel to the axis of the object, the radiation received by certain detection zones being those which are emitted by the elementary cells of the object in a direction perpendicular to the plane of the detector, the detection zones providing, for each angular position $\theta_p$, electrical signals whose amplitudes have values which depend on the intensities of radiation received, these amplitude values being stored in order to obtain the images of the object, in the cross-section planes, of ordinates k perpendicular to the axis of the object, characterized in that it consists, in order to obtain the images of the object in the cross-section planes ;

— in generating from the stored amplitude values, for each cross-section plane of ordinates k for all angular positions $\theta_p$, and all detection zones of numbers n which receive radiation, correction values for the values of these amplitudes, these correction values being obtained on initialization, from simulated amplitude values corresponding to initialization, a simulated image of the object in which it is assumed that the distribution of the tracer is uniform, and from stored correction coefficients, relating to the attenuation ($\mu$) of radiation within the object, as a function of the coordinates (i, j) of each elementary cell and relative to the geometry of each detector,

— in determining and storing for each cross-section plane, for all the angular positions ($\theta_p$) and for the detection zones of numbers n which receive radiation, the values of the respective differences between the values of the amplitudes of the signals provided by these zones and the correction values, the images corresponding to the values of the amplitudes of the signals provided by the detection zones being available for the cross-section planes for which the values of the differences satisfy a predetermined convergence criterion, then, when the values of the differences do not satisfy the predetermined criterion, the process then consisting :

— in generating other values for correcting the values of the amplitudes of the signals, for the cross-section planes of ordinates k, for all the angular positions $\theta_p$ and for all the detection zones of numbers n which receive radiation, these other correction values being obtained from the stored values of differences, from the said correction coefficients, and from stored filtering coefficients ($\lambda$) of these other correction values,

— in carrying out for each cross-section plane of ordinates k, for all the angular positions ($\theta_p$) and for all the detection zones receiving radiation, additions between the stored amplitude values and the other correction values respectively, the values of the results of these additions being stored, in order to provide the corresponding images of the object in the cross-section planes of ordinates k or to obtain new correction values, from the results of these additions, until the convergence of these results.

2. Process according to Claim 1, characterized in that the correction values are obtained for each cross-section plane of ordinates k, for all the angular position $\theta p$ and for all the zones receiving radiation, by multiplication of the values of amplitudes simulated or resulting from the additions, with the values of the correction coefficients.

3. Process according to Claim 2, characterized in that the other correction values are obtained, for each cross-section plane of ordinates k, for all the angular positions $\theta p$ and for all the zones receiving radiation, by multiplication of the said differences with the said correction coefficients and with the said filtering coefficients.

4. Process according to Claim 3, characterized in that it consists, for each cross-section plane of ordinates k, for all the angular positions $\theta p$, in determining and storing, prior to obtaining the correction values, coordinates (i, j) of a mask of the space outside the images of the desired cross-sections, in determining the correspondences between the numbers of the detection zones n and the coordinates (i, j) of the elementary cells located in the mask, then in comparing the numbers of the detection zones defined by the mask with the numbers of the detection zones receiving radiation.

5. Process according to Claim 4, characterized in that it consists in using a microcomputer device connected to at least one memory and to means for the display of images, this device also enabling the control of the rotation of the detector, the referencing of the angular positions $\theta p$ around the object, the references of the detection zones and the coordinates, i, j, k and in order to carry out all the operations of storage, correction and display of the images.

6. Device for obtaining three-dimensional images of an object from radiation emitted by a tracer contained within that object, including a detector-plane (D) having detection zones referenced by numbers n and capable of providing signals depending respectively on the intensities of radiation received in a direction perpendicular to the plane (P) of the detector, this detector being rotated around the object in such a way that it remains constantly tangential to an imaginary cylinder (C) whose axis corresponds to an axis of the object, means (13) of controlling and referencing the angular positions ($\theta p$) of the detector, these positions being referenced between a reference plane ($P_R$) passing through the axis of the object and a plane passing through the axis of the object and perpendicular to the plane of the detector, the object being divided up into imaginary elementary radiation-emitting cells referenced by their coordinates in a fixed frame of reference (i, j, k), the k axis of this frame of reference being parallel to the axis of the object, the radiation received by certain detection zones referenced by their numbers n being those which are emitted by the elementary cells of the object in a direction perpendicular to the

12

plane of the detector, the detection zones providing, for each angular position θp, electrical signals whose amplitudes have values which depend on the radiation intensities received, these amplitude values enabling the images of the object to be obtained in the cross-section planes, of ordinates k, perpendicular to the axis of the object, the device also including iterative means (14, 15) of processing by convergence of the values of the amplitudes of the signals coming from the detection zones which have received radiation, these processing means being connected to means of display (17) in order to display the images of the object corresponding to each cross-section plane of ordinate k after each iteration, characterized in that the processing means comprise :

— means of storage (15) which, for all the ordinates k of the cross-section planes and all the angular positions θp of the detector, enable the storage of :

the values of the amplitudes of the signals provided by the detection zones of numbers n which have received radiation,

the coordinates (i, j) of the elementary cells capable of having emitted radiation towards these detection zones of numbers n,

signal amplitude reference values, these values being simulated or obtained after a prior processing by the device of the values of the signals corresponding to the detection zones,

correction coefficients to be applied to the reference values,

at least one filtering coefficient of the amplitude values,

— first means of multiplication (22) of the reference amplitude values with the correction coefficients, the values of the results of these multiplications being recorded by the means of storage (15),

— means (23) of comparing the values of the amplitudes of the signals provided by the detection zones with the reference values, the values of the results of these differences being recorded in the means of storage (15),

— means of testing (24) the convergence of the values of the differences according to a predetermined convergence criterion, the means of display (17) being connected to the means of testing (24) in order to display the images of the cross-sections of ordinates k when the convergence criterion is satisfied,

— second means of multiplication (26) of the values of the differences with the correction coefficients and the filtering coefficient, these means intervening when the convergence criterion is not satisfied, the values of the results of these multiplications being recorded in the means of storage (15),

— means of adding (27) the values of the results of the multiplications with the values of the reference amplitudes, the means of testing (24) being connected to the means of display (17) in order to control the display of the images of the cross-sections of ordinates k from the results of the additions, when a convergence criterion of the values of the results of the additions is satisfied, these means of addition being connected to the first means of multiplication (22) and to the means of storage (15) in order that, when the convergence criterion of the values of the results of the additions is not satisfied, the reference values provided to these second means of multiplication (22) are the values of the results of the additions.

7. Device according to Claim 6, characterized in that it also includes means of control (16) of the means of storage (15) in order that these means record, for each ordinate k of the cross-section plane, and for all the angular positions θp, masking coordinates (i, j) defining a limit outline for the reconstruction of the image of the object, and means (28) of comparing the masking coordinates with the coordinates (i, j) of the elementary cells having emitted radiation, in order to take into account only the detection zones whose numbers n correspond to elementary cells located within the masking limit outline.

8. Device according to Claim 7, characterized in that the correction coefficients recorded in the means of storage (15) relate to the geometry of the detection zones, to the solid angles at which the detection zones receiving radiation are seen from the corresponding elementary cells of the object, and to the attenuation of radiation as a function of the distance separating an elementary cell from the masking outline.

9. Application of the process and of the device according to any one of Claims 1 to 8 to the tomographic reconstruction of three-dimensional images of an organ in which a tracer emitting gamma radiation has been injected.

**Patentansprüche**

1. Verfahren für die dreidimensionale Wiedergabe eines Objekts anhand von Strahlungen, die von einem Indikator abgegeben werden, der in diesem Objekt enthalten ist, wobei die Strahlungen von mehreren Detektorzonen einer Detektorebene empfangen werden, die durch Nummern (n) in dieser Ebene des Detektors markiert sind, dieser Detektor in eine Drehbewegung um das Objekt versetzt ist derart, daß er ständig einen fiktiven Drehzylinder (C) berührt, dessen Achse einer Achse des Objekts entspricht, die Winkelpositionen der Detektorebene durch Winkel $\theta_p$ zwischen einer Bezugsebene, die durch die Achse des Objekts verläuft, und einer Ebene bestimmt sind, die durch die Achse des Objekts verläuft und auf der Detektorebene senkrecht steht, das Objekt fiktiv in Strahlungsemissionselementarzellen aufgeteilt ist, die durch ihre Koordinaten in einem Bezugspunkt (i, j, k) bestimmt sind, wobei die Achse k durch diesen Bezugspunkt parallel zur Achse des Objekts ist, die durch gewisse Detektorzonen empfangenen Strahlungen solche sind, die von den Elementarzellen des Objekts in einer Richtung ausgesandt werden, die senkrecht zur Detektorfläche ist, die Detektorzonen für jede Winkelposition $\theta_p$ elektrische Signale liefern, deren Amplituden Werte haben, die von den Intensitäten der empfangenen Strahlungen abhängen, diese Amplitudenwerte gespeichert werden, um die Abbildungen des Objekts in Schnittebenen von Koordinaten k senkrecht zur Achse des Objekts zu erhalten, dadurch gekennzeichnet, daß es zur Erzielung der Abbildungen des Objekts in den Schnittebenen enthält :

— Erzeugen anhand der gespeicherten Amplitudenwerte für jede Schnittebene von Koordinaten k für alle Winkelpositionen $\theta_p$ und für alle Detektorzonen der Nummer n, die die Strahlungen empfangen, von Korrekturwerten für die Werte dieser Amplituden, wobei diese Korrekturwerte bei der Initialisierung erhalten werden mit Hilfe von simulierten Amplitudenwerten, die bei der Initialisierung einem simulierten Abbild des Objekts entsprechen, von dem angenommen wird, daß die Verteilung des Indikators darin gleichförmig ist, und mittels gespeicherten Korrekturkoeffizienten, die sich auf die Dämpfung ($\mu$) der Strahlungen in dem Objekt beziehen, in Funktion der Koordinaten (i, j) jeder Elementarzelle, und die sich auf die Geometrie eines jeden Detektors beziehen,

— Ermitteln und Speichern für jede Schnittebene, für alle Winkelpositionen ($\theta_p$) und für alle Detektorzonen der Nummern n, die Strahlungen empfangen, der Werte der entsprechenden Differenzen zwischen den Amplitudenwerten der von diesen Zonen gelieferten Signale und den Korrekturwerten, wobei die Abbilder, die den Amplitudenwerten der von den Detektorzonen gelieferten Signalen entsprechen, für die Schnittebenen zur Verfügung stehen, für welche die Differenzwerte auf ein vorbestimmtes Konvergenzkriterium ansprechen, und sodann, wenn die Differenzwerte nicht auf das vorbestimmte Kriterium ansprechen, das Verfahren dann wie folgt abläuft :

— Erzeugen anderer Korrekturwerte der Signalamplitudenwerte für die Schnittebenen der Koordinaten k für alle Winkelpositionen $\theta_p$ und für alle Detektorzonen der Nummern n, die die Strahlungen empfangen, wobei man diese anderen Korrekturwerte mit Hilfe von gespeicherten Differenzwerten anhand der genannten Korrekturkoeffizienten und anhand von gespeicherten Filterkoeffizienten ($\lambda$) dieser anderen Korrekturwerte erhält,

— Erzeugen für jede Schnittebene der Koordinaten k für alle Winkelpositionen ($\theta_p$) und für alle Detektorzonen, die Strahlungen empfangen, von Additionen zwischen jeweils den gespeicherten Amplitudenwerten und den anderen Korrekturwerten, wobei die Werte dieser Additionsergebnisse gespeichert werden, um die entsprechenden Bilder des Objekts in den Schnittebenen der Koordinaten k zu liefern oder um neue Korrekturwerte mit Hilfe dieser Additionsergebnisse zu erhalten, bis zur Konvergenz dieser Resultate.

2. Verfahren nach Anspruch 1, dadurch gekennzeichnet, daß die Korrekturwerte für jede Schnittebene von Koordinaten k für alle Winkelpositionen $\theta_p$ und für alle Strahlungen empfangenden Zonen durch Multiplikation der simulierten Amplitudenwerte oder der Additionsergebnisse mit den Werten der Korrekturkoeffizienten erhalten werden.

3. Verfahren nach Anspruch 2, dadurch gekennzeichnet, daß die anderen Korrekturwerte für jede Schnittebene von Koordinaten k für alle Winkelpositionen $\theta_p$ und für alle Strahlungen empfangenden Detektorzonen durch Multiplikation der genannten Differenzen mit den genannten Korrekturkoeffizienten und mit den genannten Filterkoeffizienten erhalten werden.

4. Verfahren nach Anspruch 3, dadurch gekennzeichnet, daß es enthält : Bestimmen und Speichern für jede Koordinatenschnittebene k für alle Winkelpositionen $\theta_p$ vor der Bildung der Korrekturwerte von Koordinaten (i, j) einer Raummaske außerhalb der gewünschten Schnittbilder, Ermitteln der Korrespondenzen zwischen den Nummern der Detektorzonen n und der Koordinaten (i, j) der Elementarzellen, die in der Maske liegen, anschließend Vergleichen der Nummern der durch die Maske definierten Detektorzonen mit den Nummern der Detektorzonen, die Strahlungen empfangen.

5. Verfahren nach Anspruch 4, dadurch gekennzeichnet, daß es enthält : Verwenden einer Mikrocomputereinrichtung, die mit wenigstens einem Speicher verbunden ist, und einer Bildanzeigeeinrichtung, wobei die Mikrocomputereinrichtung weiterhin die Steuerung der Detektordrehung, die Markierung der Winkelpositionen $\theta_p$ um das Objekt, die Markierung der Detektorzonen und der Koordinaten i, j, k erlaubt und mit der alle Speicher-, Korrektur- und Bildanzeigeoperationn ausgeführt werden.

6. Gerät für die dreidimensionale Wiedergabe eines Objekts anhand von Strahlungen, die von einem Indikator ausgesandt werden, der in diesem Objekt enthalten ist, enthaltend eine Detektorebene (D), die Detektorzonen aufweist, die durch Nummern n bezeichnet sind und dazu geeignet sind, Signale zu liefern, die entsprechend von den Strahlungsintensitäten abhängen, die in einer Richtung senkrecht zur Ebene (P) des Detektors empfangen werden, wobei der Detektor in eine Rotationsbewegung um das Objekt versetzt ist derart, daß er ständig in Berührung mit einem fiktiven Zylinder (C) bleibt, dessen Achse einer Achse des Objekts entspricht, Einrichtungen (13) zum Steuern und Zuordnen der Winkelpositionen ($\theta_p$) des Detektors, wobei diese Positionen zwischen einer Bezugsebene ($P_R$), die durch die Achse des Objekts verläuft, und einer Ebene bestimmt sind, die durch die Achse des Objekts verläuft und zur Detektorebene senkrecht steht, wobei das Objekt fiktiv in Strahlungsemissionselementarzellen unterteilt ist, die durch ihre Koordinaten in einem festen Bezugssystem (i, j, k) bestimmt sind, wobei die Achse k dieses Bezugssystems parallel zur Achse des Objekts verläuft, die Strahlungen, die von gewissen Detektorzonen empfangen werden, die durch ihre Nummern n markiert sind, jene sind, die von den Elementarzellen des Objekts in einer Richtung senkrecht zur Detektorebene ausgesandt werden, die Detektorzonen für jede Winkelposition $\theta_p$ elektrische Signale liefern, deren Amplituden Werte haben, die von den empfangenen Strahlungsintensitäten abhängen, wobei diese Amplitudenwerte es erlauben, Abbildungen des Objekts in Schnittebenen der Koordinate k zu erhalten, die senkrecht zur Achse des Objekts verlaufen, wobei das Gerät weiterhin enthält : iterative Einrichtungen (14, 15) zur Verarbeitung durch Konvergenz der Amplitudenwerte der abgegebenen Signale der Detektorzonen, die Strahlungen empfangen haben, wobei diese Verarbeitungseinrichtungen mit Anzeigeeinrichtungen (17) verbunden sind, um die Abbilder des Objekts entsprechend jeder Koordinatenschnittebene k nach jeder Iteration zur Darstellung zu bringen, dadurch gekennzeichnet, daß die Verarbeitungseinrichtungen enthalten :

— Speichereinrichtungen (15), die für alle Koordinaten k der Schnittebenen und für alle Winkelpositionen $\theta_p$ des Detektors zu speichern erlauben :

die Amplitudenwerte der Signale, die von den Detektorzonen der Nummern n, die die Strahlungen empfangen haben, geliefert werden,

die Koordinaten (i, j) der Elementarzellen, die geeignet sind, die Strahlungen gegen die Detektorzonen der Nummern n ausgesandt zu haben,

die Bezugswerte für Signalamplituden, wobei diese Werte simuliert sind oder anschließend an eine vorangehende Verarbeitung von Signalwerten erhalten wurden, die den Detektorzonen entsprechen,

Korrekturkoeffizienten, die auf die Bezugswerte anzuwenden sind, wenigstens einen Amplitudenwertfilterkoeffizienten,

— erste Einrichtungen (22) zur Multiplikation der Bezugsamplitudenwerte mit den Korrekturkoeffizienten, wobei die Werte der Multiplikationsergebnisse durch die Speichereinrichtungen (15) gespeichert werden,

— Einrichtungen (23) zum Vergleichen der Signalamplitudenwerte, die von den Detektorzonen geliefert werden, mit den Bezugswerten, wobei die Werte dieser Differenzergebnisse in den Speichereinrichtungen (15) gespeichert werden,

— Einrichtungen (24) zum Prüfen der Konvergenz der Werte der Differenzen als Funktion eines vorbestimmten Konvergenzkriteriums, wobei die Anzeigeeinrichtungen (17) mit den Prüfeinrichtungen (24) verbunden sind, um die Bilder der Koordinatenschnitte k anzuzeigen, wenn das Konvergenzkriterium erfüllt ist,

— zweite Einrichtungen (26) zur Multiplikation der Differenzwerte mit den Korrekturkoeffizienten und dem Filterkoeffizienten, wobei diese Einrichtungen eingreifen, wenn das Konvergenzkriterium nicht erfüllt wird, wobei die Werte der Multiplikationsergebnisse in die Speichereinrichtungen (15) eingespeichert werden,

— Einrichtungen (27) zur Addition der Werte der Multiplikationsergebnisse mit den Werten der Bezugsamplituden, wobei die Prüfeinrichtungen (24) mit den Anzeigeeinrichtungen (17) verbunden sind, um die Anzeige der Bilder der Koordinatenschnitte k mittels der Additionsergebnisse zu steuern, wenn ein Konvergenzkriterium der Werte der Additionsergebnisse erfüllt ist, wobei die Additionseinrichtungen mit den ersten Multiplikationseinrichtungen (22) und den Speichereinrichtungen (15) verbunden sind, damit, wenn das Konvergenzkriterium der Werte der Additionsergebnisse nicht erfüllt ist, die an die ersten Multiplikationseinrichtungen (22) gelieferten Bezugswerte die Werte der Additionsergebnisse sind.

7. Gerät nach Anspruch 6, dadurch gekennzeichnet, daß es weiterhin Steuereinrichtungen (16) für die Speichereinrichtungen (15) enthält, damit jene für jede Schnittebenenkoordinate k und für alle Winkelpositionen $\theta_p$ Maskierungskoordinaten (i, j), die einen Objektabbildungsrekonstruktionsgrenzumriß definieren, einspeichern, und Einrichtungen (28) zum Vergleichen der Maskierungskoordinaten mit den Koordinaten (i, j) der Elementarzellen enthält, die Strahlungen ausgesandt haben, um nur die Detektorzonen in Betracht zu ziehen, deren Nummern n den Elementarzellen entsprechen, die in dem Maskierungsgrenzumriß liegen.

8. Gerät nach Anspruch 7, dadurch gekennzeichnet, daß die in die Speichereinrichtungen (15) eingespeicherten Korrekturkoeffizienten sich auf die Geometrie der Detektorzonen, auf die Festwinkel, unter denen die Detektorzonen gesehen werden, die von den dem Objekt entsprechenden Elementarzellen ausgehende Strahlung empfangen, und auf die Dämpfung der Strahlungen als Funktion des Abstandes, der eine Elementarzelle von dem Maskierungsumriß trennt, beziehen.

9. Anwendung des Verfahrens und des Geräts nach einem der Ansprüche 1 bis 8 auf die tomographische Rekonstruktion dreidimensionaler Abbilder eines Organs, in das man einen Indikator injiziert hat, der Gammastrahlungen abgibt.

FIG.1

1

# FIG.2a

**1** — EN FONCTION DES N° n DES ZONES DE DETECTION ,MEMORISATION DES VALEURS DES AMPLITUDES CODEES DES SIGNAUX In FOURNIS PAR CES ZONES, POUR CHAQUE COUPE k ET TOUS LES $\Theta_p$

**2** — MEMORISATION DE VALEURS DE MASQUAGE i, j (LIMITES DU VOLUME A RECONSTRUIRE). POUR CHAQUE k ET TOUS LES $\Theta_p$.

**3** — POUR CHAQUE k ET TOUS LES $\Theta_p$ MEMORISATION DU CODE DE CORRESPONDANCE ENTRE LES N° n DES ZONES DE DETECTION QUI ONT FOURNI DES SIGNAUX ET LES COORDONNEES i, j, DES CELLULES

Choix d'un autre $\Theta_p$ dans 3

NON

**4** — POUR CHAQUE k ET TOUS LES $\Theta_p$ COMPARAISON ENTRE i, j, de 3 et i, j, de 2 .i, j, de 3 DANS LE MASQUE ?

OUI

**5** — POUR CHAQUE k ET TOUS LES $\Theta_p$, POUR TOUT i, j DANS LE MASQUE.
- A L'INITIALISATION, MEMORISATION DE VALEURS D'AMPLITUDES SIMULEES - APRES INITIALISATION, VALEURS D'AMPLITUDES DE REFERENCE

(17)

(17)

**6** — POUR CHAQUE k ET TOUS LES $\Theta_p$, MEMORISATION DE COEFFICIENTS DE PONDERATION RELATIFS A LA GEOMETRIE DES ZONES DE DETECTION

**7** — POUR CHAQUE k ET TOUS LES $\Theta_p$, MEMORISATION DE COEFFICIENTS D'ATTENUATION DES RAYONNEMENTS RECUS PAR ZONES DE DETECTION, EN FONCTION DE i, j, n.

**8** — POUR CHAQUE k ET TOUS LES $\Theta_p$ MEMORISATION DE COEFFICIENTS DE PONDERATION CORRESPONDANT A ANGLE SOLIDE SOUS LEQUEL CHAQUE CELLULE i, j, VOIT CHAQUE ZONE DE DETECTION DE NUMERO n

**9** — POUR CHAQUE k ET TOUS LES $\Theta_p$, MULTIPLICATION DES VALEURS ET COEFFICIENTS 5, 6, 7, 8, POUR TOUTES LES ZONES DE DETECTION, MEMORISATION RESULTATS DES MULTIPLICATIONS

**10** — POUR CHAQUE k ET TOUS LES $\Theta_p$, ET POUR LES ZONES DE DETECTION
- A L'INITIALISATION ,DIFFERENCES ENTRE VALEURS DE 1 ET RESULTATS DE 9 - APRES INITIALISATION , DIFFERENCES ENTRE VALEURS DE REFERENCE DE 5 ET RESULTATS DE 9

(11)

2

POUR CHAQUE k ET TOUS LES $\Theta_p$, MEMORISATION DES VALEURS DES DIFFERENCES CALCULEES EN 10 — 11

(10) ---→

POUR CHAQUE k ET TOUS LES $\Theta_p$, LES VALEURS DE DIFFERENCES REPONDENT-ELLES A UN CRITERE DE CONVERGENCE PREDETERMINE ? — 12

OUI → AFFICHAGE DE L'IMAGE A PARTIR DE 5 OU 1

NON

POUR CHAQUE k ET TOUS LES $\Theta_p$ MEMORISATION DE COEFFICIENTS $\lambda$ DE FILTRAGE — 13

POUR CHAQUE k ET TOUS LES $\Theta_p$ ET TOUTES LES ZONES DE DETECTION, MULTIPLICATION DE 10 ET 13 — 14

MEMORISATION DES RESULTATS DE 14 — 15

**FIG.2b**

POUR CHAQUE k ET TOUS LES $\Theta_p$, MULTIPLICATION DES RESULTATS MEMORISES EN 15 ET DES COEFFICIENTS EN 6, 7, 8, POUR TOUTES LES ZONES DE DETECTION, MEMORISATION DES RESULTATS DE CES MULTIPLICATIONS — 16

POUR CHAQUE k, TOUS LES $\Theta_p$ ET TOUTES LES ZONES DE DETECTION, ADDITION DES VALEURS D'AMPLITUDES DE REFERENCE MEMORISEES EN 5 ET DES RESULTATS MEMORISES EN 16 — 17

(5)

(5)

18

MEMORISATION DES RESULTATS DES ADDITIONS 17

AFFICHAGE DE L'IMAGE A PARTIR DE 18

20

TRANSFERT DES RESULTATS DE 18 VERS 5

19

NON

CES RESULTATS REPONDENT-ILS A UN CRITERE DE CONVERGENCE PREDETERMINE ?

OUI

FIG. 3

FIG. 4

**FIG.5**

FIG.6